# EUROPÄISCHE PATENTSCHRIFT

(11) **EP 0 857 064 B1**
(45) Veröffentlichungstag und Bekanntmachung des Hinweises auf die Patenterteilung: **27.11.2002**
(21) Anmeldenummer: 96933433.3
(22) Anmeldetag: 30.09.1996
(51) Int. Cl.: A61K 31/195, A61K 9/20

(54) **STABILISIERTE SCHILDDRÜSENHORMONHALTIGE ARZNEIMITTEL**
STABILISED PHARMACEUTICAL PREPARATIONS CONTAINING THYROID HORMONE
PREPARATIONS PHARMACEUTIQUES STABILISEES CONTENANT DE L'HORMONE THYROIDIENNE

(30) Priorität: 27.10.1995 DE 19541128
(43) Veröffentlichungstag der Anmeldung: 12.08.1998
(73) Patentinhaber: HENNING BERLIN GMBH, D-12099 Berlin (DE)
(72) Erfinder: LAHR, Wolfgang, 10777 Berlin (DE); FRIESE, Andrea, 14197 Berlin (DE); WEICKGENANNT, Guido, 14163 Berlin (DE)
(74) Vertreter: Schwabe - Sandmair - Marx
(86) Internationale Anmeldenummer: EP9604274
(87) Internationale Veröffentlichungsnummer: WO97016178

(56) Entgegenhaltungen:
- DE-A- 2 546 474
- GB-A- 2 191 695
- US-A- 5 225 204
- CHEMICAL ABSTRACTS, vol. 121, no. 18, 31.Oktober 1994 Columbus, Ohio, US; abstract no. 213005, XP002025461 & JP 06 183 952 A (SANYO) 5.Juli 1994

## Beschreibung

Die Erfindung betrifft stabilisierte schilddrüsenhormonhaltige Arzneimittel sowie Verfahren zu ihrer Herstellung.

Schilddrüsenhormone im Sinne der vorliegenden Erfindung sind solche, die geeignet sind therapeutisch verwendet zu werden, insbesondere solche mit zwei bis vier Iodatomen im Molekül, synthetischer oder natürlicher Herkunft, wie z. B. Levothyroxin, Liothyronin, Dextrothyroxin, Triiodessigsäure, Thyroid (getrocknete Schilddrüse), Thyroglobulin, Diiodtyrosin sowie deren Salze.

Schilddrüsenhormone der vorgenannten Art werden therapeutisch bei Schilddrüsenerkrankungen unterschiedlicher Genese eingesetzt, besonders bei Schilddrüsenüber- oder -unterfunktion, Iodmangelerscheinungen und damit verbundenen Sekundärerkrankungen, aber auch zur prophylaktischen Substitution, und zwar als Monosubstanzen als auch in Kombination untereinander bzw. in Kombination zusammen mit anderen Wirkstoffen wie z. B. Iodsalzen. Besondere Bedeutung haben dabei Levothyroxin und Liothyronin erlangt, die in Form ihrer Salze oder als freie Säure verwendet werden.

Schilddrüsenhormone der genannten Art, insbesondere die arzneilich häufig verwendeten Salze, sind empfindlich gegen Temperatureinflüsse, Feuchtigkeit und Oxidation. Sie zersetzen sich durch zahlreiche Reaktionsmechanismen. Darüber hinaus reagieren sie mit vielen üblichen pharmazeutisch verwendbaren Hilfsstoffen, so daß es sich schwierig darstellt, Arzneiformen herzustellen, in denen der Wirkstoff über eine ausreichend lange Zeit, innerhalb der für Pharmazeutika geltenden Grenzen unter normalen Lagerbedingungen, d.h. ohne besondere Schutzvorkehrungen, erhalten bleibt.

Konventionelle Levothyroxin-Natrium-Tabletten sind beispielsweise unter mitteleuropäischem Normalklima (Klimazone I, d.h. einer mittleren Temperatur von 21 °C) bis zu einem noch tolerablen Gehalt von mindestens noch 90 % der deklarierten Wirkstoffmenge bis max. 3 Jahren haltbar, während die Haltbarkeit bei höheren Temperaturen und Luftfeuchtigkeiten (Klimazone II-IV) über diesen Zeitraum nicht mehr gegeben ist, was zu einer als nachteilig zu beurteilenden verkürzten Verkehrsfähigkeit führt. Auch die für Klimazone I tolerierte Gehaltsabnahme um 10 % bis zum Erreichen der Verkehrsfähigkeit muß als kritisch angesehen werden, da in der Regel über die Art und Wirksamkeit der entstandenen Zersetzungsprodukte wenig bekannt ist, besonders im Hinblick auf ihre Toxizität.

Es hat nicht an Versuchen gefehlt, Schilddrüsenhormone, insbesondere das therapiebestimmende Levothyroxin für die orale Verabreichung am Menschen in der Arzneiform zu stabilisieren. Das US-Patent 5 225 204 (Chen et al.) schlägt beispielsweise die Herstellung eines Komplexes aus Levothyroxin-Natrium zusammen mit Polyvinylpyrrolidon und einer Cellulosekomponente zur Stabilisierung vor. Ohne daß in dieser Schrift das Ausmaß der Stabilisierung näher erläutert wird, besitzt dieses Verfahren den Nachteil der aufwendigen Komplexherstellung, wobei besonders die Verwendung organischer Lösungsmittel aus Kosten- und ökologischen Gründen nachteilig zu beurteilen ist.

Aufgabe war es, schilddrüsenhormonhaltige Arzneiformen so zuzubereiten, daß deren Haltbarkeit ohne besondere Aufbewahrungsmaßnahmen über mehr als drei Jahre bei Klimazone I und mindestens 3 Jahre bei Klimazone II-IV gewährleistet und durch ein einfaches und umweltverträgliches Herstellverfahren gekennzeichnet ist.

Dabei sind nach gültiger Auffassung die Klimazonen wie folgt definiert ("Die Regelung der Arzneimittel in der Europäischen Gemeinschaft", Band III, Kommission der EG, Januar 1989):

**Tabelle 1**

| Klimabedingung | Zone I gemäßigt | Zone II mediterran (subtrop.) | Zone III heiß/trocken oder heiß/mäßige LF | Zone IV sehr heiß/feucht |
|---|---|---|---|---|
| Jahresmittel der Temperatur | < 20,5 °C | 20,5 - 24 °C | > 24 °C | > 24 °C |
| kinetische Mitteltemperatur (virtuelle Temperatur) | 21 °C | 26 °C | 31 °C | 31 °C |
| Jahresmittel der relativen Luftfeuchtigkeit | 45 % | 60 % | 40 % | 70 % |

Für die Länder der Europäischen Gemeinschaft treffen die Zonen I und II zu. (LF=Luftfeuchtigkeit)

Es wurde gefunden, daß das als Antagonist bei Cyanidvergiftungen medizinisch verwendete Natriumthiosulfat geeignet ist, Schilddrüsenhormonpräparationen derart zu stabilisieren, daß deren Stabilität gegenüber konventionellen Zubereitungen in nicht vorhersehbarer Weise erhöht ist. Natriumthiosulfat ist im Sinne der Toxizität als nicht toxisch anzusehen. Laut ADI-Liste (acceptable daily intake) ist die tägliche Einnahme von bis zu 700 µg/kg Körpergewicht zulässig, was für einen ca. 70 kg schweren Erwachsenen einer Menge von bis zu 49 mg pro Tag entsprechen würde (Martindale, The Extra Pharmacopoeia, London 1982, Seite 392-393).

Es wurde gefunden, daß ein Massenverhältnis von dem zu stabilisierenden Schilddrüsenhormon zu Natriumthiosulfat im Bereich von 1:0,1 bis 1:50 geeignet ist, den stabilisierenden Effekt auszuüben, wobei die empfohlene Obergrenze von ungefähr 50 mg Natriumthiosulfat als Tageshöchstdosis nicht überschritten wird.

Da Schilddrüsenhormone patientenindividuell nach dem Ausmaß der Erkrankung dosiert werden, wie z. B. das Levothyroxin von in der Regel unter 25 bis etwa 300 µg pro Darreichungsform und Tag für die Dauertherapie und bis zu 1 mg für diagnostische Zwecke, schwanken die absoluten Mengen an stabilisierendem Natriumthiosulfat zwangsläufig, wobei die untere Grenze von 1:0,1 zweckmäßigerweise bei den höheren Dosierungen und die obere Grenze von 1:50 bei niedrigeren Dosierungen praktisch angewendet wird.

Eine besondere Ausgestaltung des erfindungsgemäßen Gegenstandes ist, daß das Natriumthiosulfat in eine Matrix eingebracht wird, die im wäßrigen Medium gelöst oder suspendiert, schwach sauer bis schwach alkalisch in einem pH-Bereich von 5,5-9, vorzugsweise schwach alkalisch reagiert, um das Natriumthiosulfat selbst vor Zersetzung zu schützen. Die Matrix von Arzneiformen besteht in der Regel aus Hilfsstoffen, wie zum Beispiel Verdünnungsmitteln, Bindemitteln, Sprengmitteln, Fließregulierungs-, Schmierund Gleitmitteln, gegebenenfalls Geschmackskorrigenzien, Konservierungsmitteln, Farbstoffen oder filmbildenden Substanzen, von denen einige schwach sauer reagierende Verunreinigungen besitzen können, die auf das Natriumthiosulfat zersetzend wirken können.

Als matrixbildende Hilfsstoffe zur Formulierung schilddrüsen(hormon)haltiger Arzneiformen kommen solche zur Verwendung, die pharmakologisch und toxikologisch unbedenklich sind und wie sie z. B. in einschlägigen Arzneibüchern oder im "Handbook of Pharmaceutical Excipients" der American Pharmaceutical Association/Pharmaceutical Society of Great Britain, 1986 oder bei H.P.Fiedler, "Lexikon der Hilfsstoffe für Pharmazie, Kosmetik und angrenzende Gebiete", Editio Cantor Verlag, Aulendorf 1989, beschrieben sind. Eine Auswahl der potentiell verwendbaren Hilfsstoffe stellen die in den erfindungsgemäßen Zusammensetzungen verwendeten Hilfsstoffe dar, wie Lactose, mikrokristalline Cellulose und andere Cellulosen und Cellulosederivate, Stärke verschiedenen Ursprungs und Stärkederivate bzw. speziell behandelte Stärken, hochdisperses Siliciumdioxid sowie übliche Gleit- und Schmiermittel. Einige von diesen, z. B. Stärke, können schwach sauer reagierende Verunreinigungen besitzen, die auf das erfindungsgemäß verwendete Natriumthiosulfat zersetzend wirken, insbesondere dann, wenn bei der Herstellung der Arzneiform Wasser verwendet wird, beispielsweise bei der Feuchtgranulierung einer Tablettenmischung. In einem solchen Fall wird der Matrix eine die sauren Bestandteile neutralisierende Komponente beigefügt. Als derartige Komponente eignen sich weitere physiologisch unbedenkliche Hilfsstoffe wie Puffersubstanzen, aber auch Alkalisalze wie Natriumcarbonat oder geringe Mengen Laugen, wie z. B. Natronlauge. Da die Menge der neutralisierend und/oder puffernd wirkenden Komponente von der Menge saurer Verunreinigungen der übrigen Hilfsstoffe abhängig ist, muß der Bedarf experimentell bestimmt werden, damit sich nach Zugabe ein pH-Wert einstellt, der im wäßrigen Medium der darin gelösten oder suspendierten Matrix 5,5-9, vorzugsweise pH 6-8,5 beträgt.

Die erfindungsgemäße Verwendung von Natriumthiosulfat zur Stabilisierung von Schilddrüsenhormonen der eingangs genannten Art in Arzneimitteln, ermöglicht einfache Herstellverfahren. Diese bestehen darin, daß das Natriumthiosulfat in gelöstem Zustand der wirkstoffhaltigen Matrixmischung zugefügt wird, um eine innige Vermischung zu erreichen. Dies ist umso wichtiger, da sowohl Wirkstoff als auch der Stabilisator sehr niedrig dosiert sind.

Zur Herstellung von Tablettengranulaten geht man beispielsweise so vor, daß in der zum Granulieren erforderlichen Wassermenge das Natriumthiosulfat, gegebenenfalls zusammen mit der neutralisierend wirkenden Komponente gelöst wird und damit die übrigen Formulierungsbestandteile granuliert werden oder man gibt in die Granulierungsflüssigkeit zusätzlich das Schilddrüsenhormon und granuliert damit die Hilfsstoffmischung.

Nach üblichen Trocknungs- und gegebenenfalls Zerkleinerungsmethoden kann die Weiterverarbeitung zur gewünschten Arzneiform erfolgen, z. B. zu Tabletten, überzogenen Tabletten oder kann in Kapseln abgefüllt werden. Flüssige Formen können gleichermaßen so stabilisiert werden und z. B. als Lösung oder daraus hergestelltes Lyophilisat angeboten werden.

Anhand nachfolgender beispielhafter Zusammensetzungen wird die Erfindung erläutert, ohne den Umfang der Möglichkeiten dadurch einzuschränken. Die beispielhaft verwendeten Bestandteile entsprechen ausnahmslos Arzneibuchqualität.

### Beispiel 1

| | |
|---|---|
| Levothyroxin-Natrium | 0,60 g |
| Prägelatinierte Stärke | 225,00 g |
| Maisstärke | 327,00 g |
| Mikrokristalline Cellulose | 334,63 g |
| Natriumthiosulfat | 3,00 g |
| Natriumcarbonat | 0,77 g |
| Hydriertes Rizinusöl | 4,50 g |
| Hochdisperses Siliciumdioxid | 4,50 g |

Es wird eine Lösung, bestehend aus Wasser, Natriumthiosulfat und Natriumcarbonat hergestellt, in der das Levothyroxin suspendiert wird. Mit dieser Suspension wird eine Mischung aus mikrokristalliner Cellulose und den beiden Stärken granuliert, getrocknet, zerkleinert und mit den beiden restlichen Hilfsstoffen vermischt. Diese Mischung eignet sich zum Abfüllen in Hartgelatinekapseln oder zur Verpressung von Tabletten wählbaren Wirkstoffgehaltes. Eine Tablette mit einem beispielhaften Gewicht von 150 mg enthält dabei 100 µg Wirkstoff. Dabei ist das Massenverhältnis von Levothyroxin zu Natriumthiosulfat 1:5. Die Aufschlämmung einer derartigen Tablette in 30 ml destilliertem Wasser ergibt einen pH-Wert von 8,1.

### Beispiel 2

| | |
|---|---|
| Levothyroxin-Natrium | 0,125 g |
| Prägelatinierte Stärke | 37,500 g |
| Maisstärke | 54,250 g |
| Mikrokristalline Cellulose | 52,125 g |
| Natriumthiosulfat | 2,500 g |
| Hydriertes Rizinusöl | 0,750 g |
| Hochdisperses Siliciumdioxid | 0,750 g |

Die Herstellung erfolgt mit Wasser analog Beispiel 1. Eine Tablette mit einem Gewicht von 148 mg enthält dabei 125 µg Levothyroxin-Natrium, wobei dessen Massenverhältnis zu Natriumthiosulfat 1:20 beträgt.

### Beispiel 3

Analog der Zusammensetzung aus Beispiel 2 wurden 90 kg eines Granulates mit einem Gehalt an Levothyroxin-Natrium von 50 µg und einem Gehalt an Natriumthiosulfat von 500 µg pro 150 mg Granulat hergestellt und daraus Tabletten mit einem Durchschnittsgewicht von 150 mg gepreßt. Das Verhältnis Levothyroxin zu Natriumthiosulfat betrug 1:10.

### Beispiel 4: (Vergleich)

Analog Beispiel 3 wurden 90 kg eines Granulates ohne Natriumthiosulfat hergestellt und zu Tabletten verpreßt.

### Beispiel 5

| | |
|---|---|
| Levothyroxin-Natrium | 0,100 g |
| Liothyronin-Natrium | 0,010 g |
| Maisstärke | 92,000 g |
| Mikrokristalline Cellulose | 54,900 g |
| Natriumthiosulfat | 0,500 g |
| Natriumcarbonat | 0,900 g |
| Hydriertes Rizinusöl | 0,750 g |
| Hochdisperses Siliciumdioxid | 0,750 g |

Die Herstellung eines Granulates unter Verwendung von Wasser erfolgte analog Beispiel 1. Tabletten mit einem Gewicht von 150 mg enthalten 100 µg Levothyroxin-Natrium, 10 µg Liothyronin-Natrium sowie 500 µg Natriumthiosulfat, woraus sich ein Verhältnis Schilddrüsenhormon zu Natriumthiosulfat von 1:4,5 ergibt.

Eine in 100 ml Wasser aufgeschlämmte Tablette ergab einen pH-Wert von 7,9.

### Beispiel 6

Tabletten der Beispiele 3 und 4 wurden in handelsüblichen Blistern unter Verwendung einer 250 µm starken PVC-Folie als Bodenfolie und einer 20 µm starken Aluminiumfolie als Deckfolie verpackt und vergleichend auf ihre Stabilität geprüft. Die Lagerung erfolgte unter folgenden definierten Bedingungen:
21 °C / 45 % relative Feuchte
26 °C / 60 % relative Feuchte
30 °C / 70 % relative Feuchte
40 °C / 75 % relative Feuchte

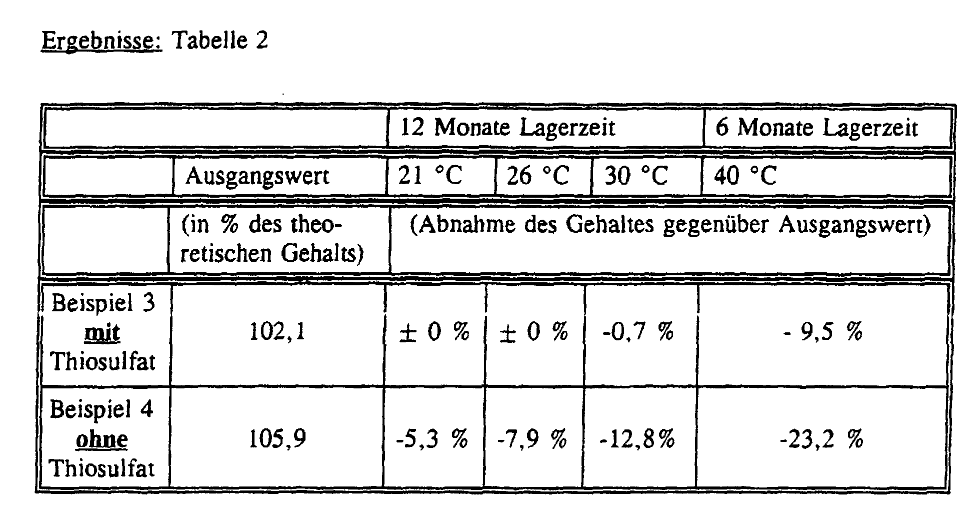

Die Gehaltswerte wurden mittels einer selektiven Analysenmethode bestimmt. Die Meßergebnisse zeigen einen dramatischen Unterschied in der Abnahme des Gehaltes an intakter Wirksubstanz und in anschaulicher Weise den stabilisierenden Effekt des Natriumthiosulfates auf das Schilddrüsenhormon.

Damit ist es nach der erfindungsgemäßen neuen Lehre möglich, mit einem einfachen Verfahren Schilddrüsenhormonpräparate herzustellen, die selbst unter extremen, ungünstigen Klimabedingungen eine so hohe Stabilität besitzen, daß eine Lagerung ohne besondere Schutzmaßnahmen möglich ist und dennoch der annähernd volle Wirkstoffgehalt über deutlich mehr als 3 Jahre zur Therapie erhalten bleibt.

## Patentansprüche

1. Stabile schilddrüsenhormonhaltige Arzneimittel, **dadurch gekennzeichnet, daß** sie Natriumthiosulfat als stabilisierende Komponente in einem Masseverhältnis von Schilddrüsenhormon zu Natriumthiosulfat von 1:0,1 bis 1:50 enthalten.

2. Arzneimittel nach Anspruch 1, **dadurch gekennzeichnet, daß** sie zusätzlich eine alkalische Komponente enthalten, deren Masse so eingestellt wird, daß die in Wasser aufgeschlämmte oder gelöste Arzneiform einen pH-Wert von 5,5 - 9 aufweist.

3. Arzneimittel nach Anspruch 2, **dadurch gekennzeichnet, daß** die alkalische Komponente ein Alkalisalz oder eine Lauge ist.

4. Arzneimittel nach Anspruch 3, **dadurch gekennzeichnet, daß** das Alkalisalz Natriumcarbonat ist.

5. Arzneimittel nach Anspruch 1, **dadurch gekennzeichnet, daß** das Schilddrüsenhormon Levothyroxin ist.

6. Arzneimittel nach Anspruch 1, **dadurch gekennzeichnet. daß** das Schilddrüsenhormon Liothyronin ist.

7. Arzneimittel nach Anspruch 1, **dadurch gekennzeichnet, daß** das Schilddrüsenhormon Diiodtyrosin ist.

8. Arzneimittel nach Anspruch 1, **dadurch gekennzeichnet, daß** das Arzneimittel mindestens zwei Schilddrüsenhormone enthält.

9. Verfahren zur Herstellung von Arzneimitteln nach einem der Ansprüche 1 bis 8, **dadurch gekennzeichnet daß** Natriumthiosulfat und gegebenenfalls eine alkalische Komponente in Wasser gelöst zugesetzt wird.

## Claims

1. Stable medicines containing thyroid gland hormone, **characterized in that** they contain sodium thiosulphate as a stabilising component in a ratio by mass of thyroid hormone to sodium thiosulphate of 1 : 0.1 to 1 : 50.

2. Medicines according to Claim 1, **characterized in that** in addition they contain an alkaline component whose mass is adjusted such that the presentation of the medicine slurried or dissolved in water has a pH of 5.5 - 9.

3. Medicines according to Claim 2, **characterized in that** the alkaline component is an alkali salt or an alkaline liquor.

4. Medicines according to Claim 3, **characterized in that** the alkali salt is sodium carbonate.

5. Medicines according to Claim 1, **characterized in that** the thyroid gland hormone is laevo-thyroxine (thyroiodine).

6. Medicines according to Claim 1, **characterized in that** the thyroid gland hormone is liothyronine (triiodothyronine).

7. Medicines according to Claim 1, **characterized in that** the thyroid gland hormone is diiodotyrosine (diiodothyronine).

8. Medicines according to Claim 1, **characterized in that** the medicine contains at least two thyroid gland hormones.

9. Method for manufacturing medicines according to one of the Claims I to 8, **characterized in that** sodium thiosulphate and optionally an alkaline component dissolved in water are added.

## Revendications

1. Préparation pharmaceutique stabilisée contenant de l'hormone thyroïdienne, **caractérisée en ce qu'**elle contient du thiosulfate de sodium comme composant stabilisant dans un rapport en masse d'hormone thyroïdienne sur thiosulfate de sodium de 1 : 0,1 à 1 : 50.

2. Préparation pharmaceutique selon la revendication 1, **caractérisée en ce qu'**elle contient en outre un composant alcalin, dont la masse est ajustée de manière à ce que la forme médicamenteuse mise en suspension ou dissoute dans l'eau présente une valeur de pH de 5,5-9.

3. Préparation pharmaceutique selon la revendication 2, caractérisée en ce le composant alcalin est un sel de métal alcalin ou une solution alcaline.

4. Préparation pharmaceutique selon la revendication 3, **caractérisée en ce que** le sel de métal alcalin est le carbonate de sodium.

5. Préparation pharmaceutique selon la revendication 1, **caractérisée en ce que** l'hormone thyroïdienne est la lévothyroxine.

6. Préparation pharmaceutique selon la revendication 1, **caractérisée en ce que** l'hormone thyroïdienne est la liothyronine.

7. Préparation pharmaceutique selon la revendication 1, **caractérisée en ce que** l'hormone thyroïdienne est la diiodothyrosine.

8. Préparation pharmaceutique selon la revendication 1, **caractérisée en ce que** la préparation contient au moins deux hormones thyroïdiennes.

9. Procédé pour la préparation d'une préparation pharmaceutique selon une des revendications 1 à 8, **caractérisé en ce qu'**on ajoute du thiosulfate de sodium et éventuellement un composant alcalin dissous dans l'eau.
